# EUROPEAN PATENT APPLICATION

(11) **EP 2 669 363 A1**
(43) Date of publication of application: **04.12.2013**
(21) Application number: 11857038.1
(22) Date of filing: 12.07.2011
(51) Int. Cl.: C12M 1/02

(54) **DUAL BODY CIRCULATORY REACTOR FOR CELLULOSE ENZYMOLYSIS AND APPLICATION THEREOF**

(30) Priority: 24.01.2011 CN 201110025868
(71) Applicant: Shanghai Zhongwei Biochemistry Co., Ltd, Shanghai 201203 (CN)
(72) Inventor: ZHANG, Shichu, Pudong District Shanghai 201203 (CN); WANG, Hengxin, Pudong District Shanghai 201203 (CN)
(74) Representative: Noel, Chantal Odile
(86) International application number: PCT/CN2011/077086
(87) International publication number: WO 2012/100510

(57) **Abstract**

The present invention provides a dual body circulatory reactor for cellulose enzymolysis and an application thereof. Specifically, the present invention provides a reactor for cellulose enzymolysis comprising: (a) a first reaction tank comprising a tank body, a shear homogenizer provided in the tank body, an inlet for feeding, and an outlet for discharging a material; (b) a second reaction tank comprising two or more reaction tubes connected in series or in parallel; (c) a pipeline connecting the first and the second reaction tanks; and (d) a pipeline connecting each tube bodies in the second reaction tank. When conducting enzyme saccharification on bast fiber crops and other celluloses, the enzymolysis reactor of the present invention has advantages of high conversion rate, high concentration, short conversion time, low enzyme consumption, and low energy consumption, and thus is especially suitable for a large volume cellulose enzymolysis reaction system.

## Description

### FIELD OF THE INVENTION

The present invention relates to a new energy field, and more specifically, relates to the saccharification field using plant cellulose as a raw material through enzymolysis. The present invention provides a dual body circulation reactor for cellulose enzymolysis using fiber of bast fiber crops as raw material and the uses thereof, and said reactor is highly efficient, of high capacity, and low energy consumption.

### BACKGROUND OF THE INVENTION

At present, the demand of mankind for energy is very large, but the resources such as oil resources are non-renewable, therefore efforts are made for developing various kinds of new energy.

Ethanol itself is combustible, and can also be used as a good petrol oxygen-increasing agent and a blending component of petrol with high octane number, therefore, it has been recognized as one of the most promising renewable and clean energy sources. The production and application of ethanol are developing rapidly in the world. The fuel ethanol industry has become important policy mean and economic lever for solving the problem of shortage of oil resources, protecting urban atmospheric environmental quality and regulating the contradiction between the supply and demand of the agricultural products. It is conservatively estimated that the demand for fuel ethanol in China will reach 20 million tons every year.

Currently, the ethanol fermentation industry mainly use food crops such as starch corn, wheat comprising starch and the like as raw materials, therefore, human and livestock will compete for food, and the cost of raw material accounts for a high proportion in the total cost. At present, for the production of biomass fuel using food crops as raw material, there is no resource condition for the further expansion due to the shortage of food resources. Producing fuel ethanol from natural cellulose materials by biological conversion not only can provide a considerable amount and economically viable new energy, but also can substantially reduce ecological environment pollution resulted from agricultural waste and thus has important economic and ecological significance.

Hydrolyzing cellulose to fermentable sugars, i.e. completing the process of saccharification of cellulose materials through enzymolysis, is important for using natural cellulose materials to produce fuel ethanol.

Cellulose is the main ingredient of plant cell wall, and is a linear long chain macromolecule formed by 8000 to 12,000 β-D-glucopyranosyl monomers through β-1,4-glycosidic linkage, wherein glucose subunits arrange closely. Meanwhile, there are intermolecular amorphous regions irregularly arranged. Cellulose usually is surrounded by hemi-cellulose and lignin, thus forming a complex even more difficult to be decomposed. Therefore, cellulose is insoluble in water, and difficultly hydrolyzed.

Cellulose can be hydrolyzed by chemical or biological method. Biological method, i.e. enzyme hydrolysis process, is considered the most promising process due to mild reaction conditions and little or no byproduct. In the recent two decades, microbial fermentation method has been favored by biologists around the world, which uses the sugar obtained through enzymatic hydrolysis of cellulose as the fermentable sugar in fermentation, thus producing ethanol. Due to low cost, simple equipment, and environmental protection, the process producing ethanol by bio-transforming natural cellulose materials has a good prospect.

The degradation process of cellulose by cellulase begins from its adsorption on the cellulose. The degradation process can be completed by a single enzyme, such as a cellulase complex formed from a variety of components in Clostridium themocellum, or collaboration of multiple enzymes. Due to the complexity of the cellulose structure, there is no enzyme in fungi that can completely hydrolyze cellulose. Whether in cellulose enzyme complex, or in fungi body, the degradation of cellulose requires the collaboration of multiple enzymes. The fungus degrades cellulose by free cellulase secreted to the extracellular environment through the hydrolase mechanism; while bacterial cellulase works by forming multi-enzymes complex.

At present, the most widely accepted mechanism of the degradation of cellulose through enzymolysis is the collaboration mechanism of endoglucanase, exoglucanase and β-glycosidase. The mechanism involves: randomly hydrolyzing β-dextran glucoside bond in cellulose molecular chains by endoglucanase, thus forming new chain ends; continuously cutting and releasing soluble cellobiose by dextran exoglycosidase from one end of cellulose molecular chain; and hydrolyzing the intermediates - cellobioses and fiber oligosaccharides by β-glycosidase to small molecules to remove their product inhibition on endoglucanase and exoglucanase. The three hydrolysis processes can be carried out simultaneously and completely degrade cellulose to glucose.

The structure of natural cellulose material is very complex. High crystallinity and lignification of the cellulose prevent the enzyme from contacting the cellulose, thus making the cellulose difficult to be directly biodegraded. Currently, for the majority of natural cellulose materials, hydrolysis rate is generally very low (< 20%), if the material is directly hydrolyzed by enzymes without suitable pretreatment.

Enzymatic fermentation methods for producing ethanol include direct fermentation, indirect fermentation, mixed-strain fermentation, simultaneous saccharification and fermentation, non isothermal simultaneous saccharification and fermentation (NSSF), and immobilized cell fermentation, etc. Among them, the simultaneous saccharification and fermentation method is a common method for bio-conversion of ethanol due to the advantages, such as time saving, high efficiency, and economy.

In a class of existing method, lignocellulose, the representative of which is crops straw, is used as the raw material to produce fuel ethanol. However, as agricultural waste, the straw, the cellulose content of which is low, distributes dispersedly, and specific volume thereof is very low so that it is difficult to collect and transport, thereby increasing the cost of raw materials. Usually it takes 6 to 7 tons of straws to produce 1 ton of ethanol. The raw material cost will greatly influence the total cost.

The fiber of bast fiber crops is one kind of raw material with very high cellulose content, therefore, bast fiber has the potential to be an important raw material for the new energy, new polymer materials and articles thereof in the furture. In addition, some bast fiber crops can grow in unused lands, such as saline-alkali land, desert and the like, with rapid growth and high yield, which would be suitable for industrialization. Kenaf, for example, is a fast-growing and high-yielding crop with strong adaptability, the stem of which is about 1.5-3.5 m in height and 1-3 cm in diameter. Such crop can be harvested after growing for 5 to 7 months with 16-20 t/hm² of yield. However, because the crystallinity, degree of polymerization, and degree of orientation of bast fiber are much higher than those of other biomass (e.g., starch), the enzymolysis process, the conversion target of which is glucose (or other monomer), is very difficult. Now, there are no satisfactory and efficient equipment and process possessing the advantages, such as little time-consuming, low energy consumption, and less consumption of enzymes for the saccharification of bast fiber through enzymolysis.

Therefore, it is urgent to develop an equipment and process for efficiently saccharifying bast fiber through enzymolysis in this field.

### SUMMARY OF THE INVENTION

The subject of the present invention is to provide an efficient large equipment and industrialization process for the saccharification of bast fiber through enzymolysis, which have the advantages of little time-consuming, low energy consumption, and less consumption of enzyme.

In the first aspect of the present invention, a reactor for cellulose enzymolysis is provided, comprising:
(a) a first reaction tank comprising a tank body, a shear homogenizer located in the tank body, an inlet for feeding, and an outlet for discharging a material;
(b) a second reaction tank comprising two or more reaction tubes connected in series or in parallel, wherein each reaction tube comprises a tube body, a rotating shaft and stirring impeller located in the tube body, an inlet, an outlet, and a thermal insulation layer located in the outside of the tube body; and the second reaction tank further comprises a motor for rotating the rotating shaft in the tube body;
(c) a pipeline connecting the first and the second reaction tanks, said pipeline comprises a pipeline for transferring the material processed in the first reaction tank to the second reaction tank, and a pipeline for transferring the material processed in the second reaction tank to the first reaction tank, wherein each pipeline can be equipped with an optional pipeline pump; and
(d) a pipeline connecting each tube body in the second reaction tank, wherein each pipeline can be equipped with an optional pipeline pump.

In another preferred embodiment, the volume (or cubage) of the first reaction tank is 3-150 t, and preferably 30-100 t.

In another preferred embodiment, there are at least two shear homogenizers in the tank body.

In another preferred embodiment, the number of shear homogenizers is 3-6.

In another preferred embodiment, the first reaction tank further includes a tank cap rotating around the center axis and being located on the top of the tank body, and the shear homogenizer is fixed in the tank top.

In another preferred embodiment, the rotation speed of the tank cap rotating around the center axis and being located on the top of the tank body is 1-20 rpm, and preferably 2-10 rpm.

In another preferred embodiment, the tank cap of the first reaction tank is further fixed with a tank cover.

In another preferred embodiment, the tank cap is sealed in the tank body of the first reaction tank by the tank cover.

In another preferred embodiment, the rotation speed of the tank cap rotating around the center axis is 2-50 rpm.

In another preferred embodiment, said shear homogenizers can be distributed with a staggered manner in the tank body of the first reaction tank.

In another preferred embodiment, said shear homogenizers can be uniformly distributed in a circumference at a distance of one radial distance from the center axis.

In another preferred embodiment, the multiple reaction tubes in the second reaction tank are arranged broadwise and step-wise from top to bottom.

In another preferred embodiment, the rotation speed of said rotating shaft located in the tube body in the second reaction tank can be 50-300 rpm, and preferably 100-250 rpm.

In another preferred embodiment, said rotating shaft in the second reaction tank is equipped with blades at helix angle of 15-45° to flow direction.

In another preferred embodiment, the depth-diameter aspect ratio of the second reaction tank body is 1: 0.5-1: 0.1, and preferably 1: 0.25.

In another preferred embodiment, each tube body of the first reaction tank and/or the second reaction tank also has a temperature measuring port.

In another preferred embodiment, the first reaction tank also has a thermal insulation layer located in the outside of the tube body.

In another preferred embodiment, the rotation speed of the shear homogenizer located in the first reaction tank is 500-4000 rpm, and preferably 1200-3000 rpm.

In another preferred embodiment, the ratio of the volume of the first reaction tank to that of the second reaction tank is 0.1: 1-1: 0.1, and preferably 1: 0.5, wherein the volume of the second reaction tank is the sum of the volume of each tube body.

In another preferred embodiment, the reactor further comprises a filtrator which is connected with the outlet to discharge a material for filtrating the material upon enzymolysis to obtain a clear enzymatic hydrolysate with high concentration of saccharide (≥ 10%).

In the second aspect of the present invention, a method for the saccharification of cellulose through enzymolysis is provided, said method comprises the following steps:
(a) homogenizing a cellulose raw material in the first reaction tank through shear force to form a homogenized cellulose raw material;
(b) hydrolyzing the homogenized cellulose raw material by using enzymes in the second reaction tank to degrade cellulose into oligosaccharide or monosaccharide, wherein the second reaction tank comprises two or more reaction tubes connected in series or in parallel, and in step b), the cellulose material self-circulates between or among the reaction tubes and the material exteriorly circulates between the first reaction tank and the second reaction tank.

In another preferred embodiment, the method is carried out in any one of the reactors for the enzymolysis of cellulose according to the first aspect of the invention.

In another preferred embodiment, in step a), the rotation speed for high-speed shearing is 500-4000 rpm, preferably, 1200-3000 rpm.

In the third aspect of the present invention, a use of any one of the reactors for the enzymolysis of cellulose according to the first aspect of the invention is provided, for the saccharification of cellulose raw material through enzymolysis.

In another preferred embodiment, said cellulose raw material includes bast fiber (e.g. kenaf).

It should be understood that, within the scope of the present invention, the technical features specifically described above and below (such as the Examples) can be combined with each other, thereby constituting a new or preferred technical solution which needs not be described one by one.

### DESCRIPTION OF THE DRAWINGS

Fig. 1 is a whole structure view of the equipment for the saccharification through enzymolysis in an example of the invention;
Fig. 2 is a front view of the equipment for the saccharification through enzymolysis in an example of the invention;
Fig. 3 is a whole structure view of large volume dual body circulatory equipment for the saccharification of cellucose through enzymolysis in another example of the invention;
Fig. 4 is a front view of the first reaction tank in another example of the invention;
Fig. 5 is a side view of the second reaction tank in another example of the invention;
Fig. 6 is a schematic diagram of the second reaction tank in another example of the invention.

### Reference signs are listed as follows.

- 1: the first reaction tank
- 10: electro-motor of the first reaction tank
- 11: inspection window of the first reaction tank
- 12: inlet of the first reaction tank
- 13: temperature measuring port of the first reaction tank
- 14: the tank body
- 15: high speed shear homogenizer of the first reaction tank
- 16: thermal insulation layer of the first reaction tank
- 17: outlet of the first reaction tank
- 18: tank cap of the first reaction tank
- 19: tank cover of the first reaction tank
- 2: the second reaction tank
- 21: thermal insulation layer of the second reaction tank
- 22: tube body of the second reaction tank
- 23: inlet of the second reaction tank
- 24: electro-motor of the second reaction tank
- 25: outlet of the second reaction tank
- 26: rotating shaft of the second reaction tank
- 27: stirring blade (or impeller) of the second reaction tank
- 3: pipeline connecting the first and the second reaction tanks
- 31, 32: pump
- 4: pipeline connecting each tube body in the second reaction tank

### SPECIFIC MODE FOR CARRYING OUT THE INVENTION

Upon extensive and in-depth research, the inventors provided a dedicated equipment for the saccharification of cellucose through enzymolysis for the first time. Using the equipment, the saccharification of cellucose, such as bast fiber and the like, through enzymolysis will possess the advantages of high conversion rate, high concentration, short conversion time, low enzyme consumption, low energy consumption, etc., thus especially suitable for large volume reaction system of cellulose enzymolysis.

Specifically, the inventors proposed the "five-keypoint" theory about the saccharification through enzymolysis by studying the respective features of bast fiber and cellulase involving in the reaction, and based on this theory, the dual body circulatory reactor for cellulose enzymolysis is designed and manufactured. Specifically, the "five-keypoint" theory includes:
"purifying"——ensuring high content of cellulose by pretreatment;
"refining"——ensuring minimization of geometry size of cellulose-based substrate by pretreatment;
"homogenizing"——ensuring the mix of cellulose-based substrate system and cellulase system in the period as short as possible during the reaction and appearing a very homogeneous fluid state in microcosmic structure;
"stablizing"——ensuring good heat transmission and the stability of the given reaction temperature in each space part of the whole solution over a long time of the reaction process;
"activating"——reducing the energy barrier of cellulose and making the cellulose obtain energy supplement required for the retention of activity over a long time of the reaction process.

### Terminology

As used herein, the term "bast fiber" refers to a fiber material from bast fiber crops and rich in cellulose, especially a fasciculate fiber from the interior bast layer of the stem of dicotyledonous plants. Examples of bast fiber crops include (but not limited to): kenaf, jute, ramie, flax, abutilon, nettle, bluish dogbane.

As used herein, the term "kenaf" (scientific name) is also known as ambary, wild hemp, ambari hemp, etc., and belongs to a dicotyledoneae malvacea hibiscus plant.

### Equipment for the saccharification through enzymolysis

The invention provides a large volume dual body circulatory reactor for the saccharification of bast fiber through enzymolysis for achieving the purpose, such as high conversion rate, high concentration, short conversion time, low enzyme consumption, and low energy consumption.

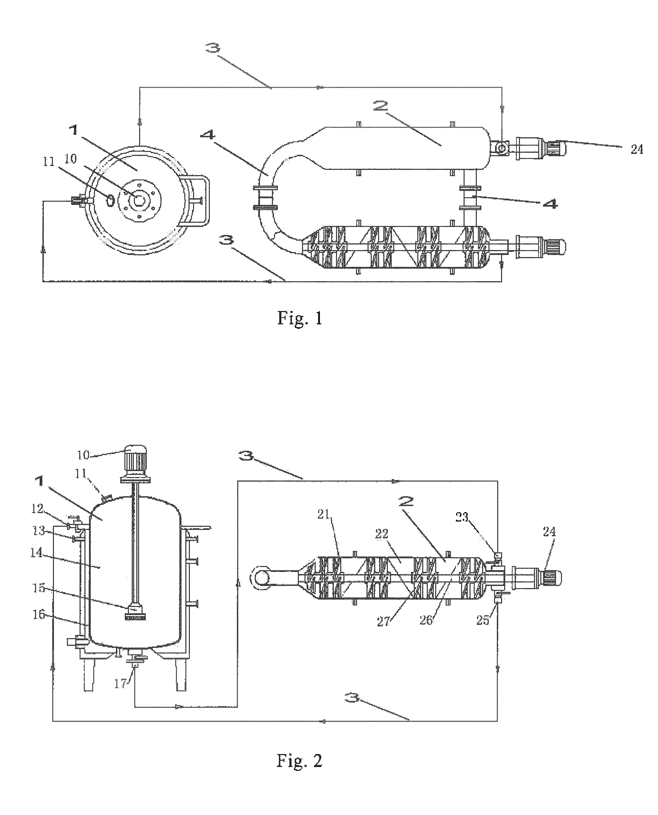

Referring to fig. 1 and fig. 2, the structure of dual body circulatory reactor for cellulose enzymolysis comprises: dual body (the first reaction tank 1 and the second reaction tank 2): the shape of the tank body of the first reaction tank 1, in which direct or inclined plug, single or multiple machine high speed shear homogenizers 15 is installed, is similar to that of conventional bucket-like enzyme reactor. The second reaction tank 2, in which the mixer comprising rotating shaft and stirring impeller is installed, is vertical or horizontal tube-like having two or more tubes. The first reaction tank and the second reaction tank are in series or in parallel. Materials enter from the underneath and discharge from the top. The dual cycle between long-range (the first reaction tank) and short-range (the second reaction tank) can be switched with high frequency through conduction, thereby greatly increasing the efficiency of enzymolysis.

The structure of the first reaction tank 1 comprises a tank body 14 (or bucket body) (can be fixed with thermal insulation layer jacket 16), a high speed shear homogenizer 15 (500-2500 rpm of rotation speed), an inlet 12, an outlet 17, a temperature measuring port 13, a sight glass or inspection window 11, a support (not shown in figures), etc..

The structure of the second reaction tank 2 comprises a tube body 22, a rotating shaft 26 (50-250 rpm of rotation speed), impeller 27, an inlet 23, an outlet 25 (self-circulation), a temperature measuring port (not shown in figures), a support, a integral thermal insulation layer 21, an electro-motor 24, etc., wherein each tube is connected by a pipeline 4, and the pipeline 4 optionally is equipped with pipeline pumps 19, 20.

The first reaction tank and the second reaction tank are connected together and multiple pipelines are connected in the second reaction tank.

In another preferred embodiment, the volume ratio between two reaction tanks is equal to or about 1: 0.5.

In the invention, the volume of dual body circulatory reactor for cellulose enzymolysis has no specific limit. Usually, the volume is at least 100 liter, more preferably more than 1 ton. The volume of the representative enzymolysis reactor can be 1-200 t, preferably, 3-150 t, more preferably, 30-100 t based on the volume of the first reaction tank.

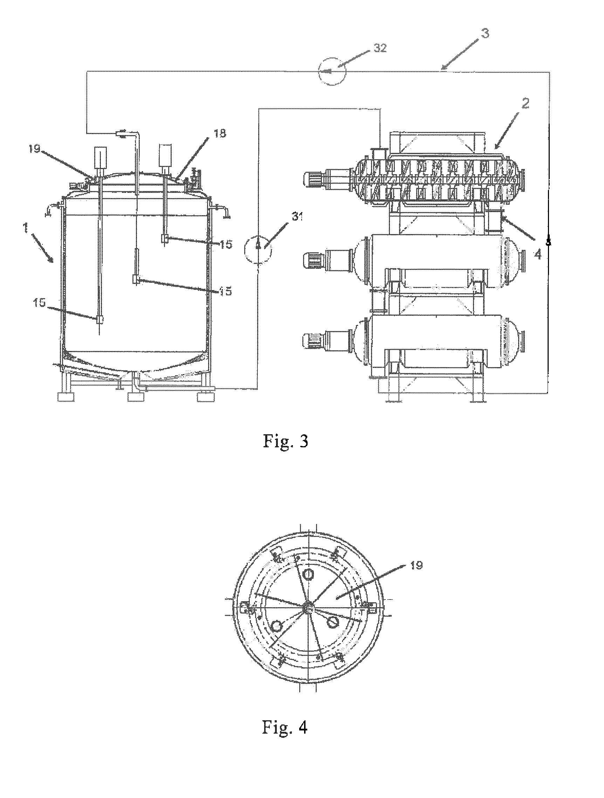

Referring to fig. 3 and fig. 4, a large volume dual body circulatory reactor for cellucose enzymolysis of the invention is shown. In the example, the structure of the second reaction tank 2 is same as that shown in the first example except the number of the second reaction tank 2 is five. Certainly, other number can be selected according to actual situation.

In the example, there is an opening on the top of the tank body 14. The first reaction tank 1 further comprises a tank cap 18 setting on the open top of the tank body 14. The tank cap 18 and the tank body 14 are arranged in the same central axis, and the tank cap 18 is configured to rotate around the central axis against the tank body 14. The mechanism for driving the rotation of the tank cap 18 can be a conventional transmission shifting mechanism driven by a motor, for example, a mechanism shown in figures. It should be understood that the transmission shifting mechanism is a well-known structure for the skilled in the art, therefore, the structure of which is not described in detail herein. It can be understood that other equivalent means can be used by the skilled in the art, in addition to the transmission mechanism and mating structure shown in the figures, so long as the tank cap 18 can be driven to rotate coaxially against the tank body 14. Therefore, these equivalent means should fall within the scope of the invention.

There is a tank cover 19 arranged above the tank cap 18, which can play a role in seal and dust-prevention, thereby making the reactor achieve better reaction effects.

In the interior of the tank body 14, three small power shear homogenizers 15 are equipped and distributed with a staggered manner. In particular, the shear homogenizers 15 are inserted into the tank body 14 to different depth through the tank cap 18, and pivotedly supported on the tank cap 18. These shear homogenizers 15 are located on a circumference at a distance of one radial distance from the center axis and separated from each other. By such structure, each shear homogenizer 15 can rotate on its own axis, and at the same time, can rotate on the central axis together with the tank cap 18, which is similar to planetary systems revolving both round the sun and on its own axis. Because in such structure, multiple small power shear homogenizers 15 are distributed with a staggered manner in the tank body 14, and besides rotating on their own axis, they can rotate together with the tank cap 18, for the solution in large volume tank, exchange can be sufficiently achieved in each spatial position, thereby greatly improving feeding quantity and the shearing homogeneity to the materials, which in turn produce the advantages, such as high conversion rate, high concentration, short conversion time, low enzyme preparation consumption, low energy consumption and so on.

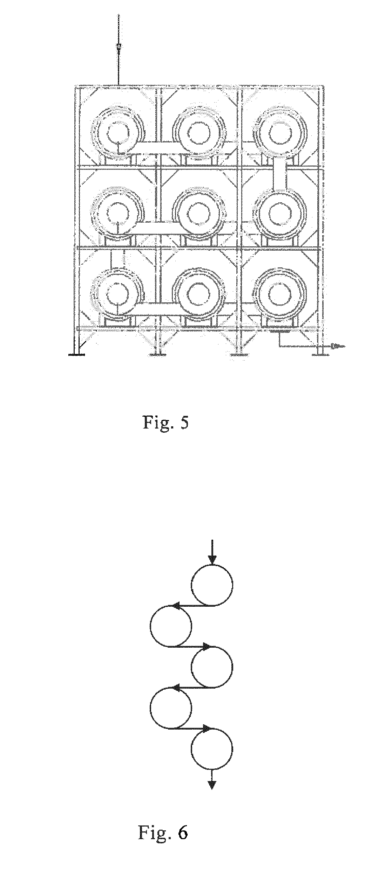

Referring to fig. 5, another example of the second reaction tank of high volume dual body circulation reactor for cellulose enzymolysis according to the invention is shown. In the example, there are 9 reaction tubes in the second reaction tank. Centainly, other number of reaction tubes can be selected according to actual situation.

Referring to fig. 6, a more preferable example of high volume dual body circulation reactor for cellulose enzymolysis of the invention is shown. This example differs from the second example in that the number of reaction tubes in the second reaction tank showed in the figure is 5, and the tubes are arranged broadwise and step-wise from top to bottom. In the second reaction tank, the materials can flow from top to bottom by potential energy. The materials are delivered to the first reaction tank by pipeline pump after they reach the bottom. In the structure, the materials is delivered by gravity, and thus the energy consumption is decreased to achieve the purpose of energy saving.

Furthermore, dual body circulation reactor for cellulose enzymolysis is optionally equipped with additional supporting facilities, including (but not limited to) filtrator for obtaining clear enzymatic hydrolysate with high concentration (more than 10%) added into the fermentation system.

### Saccharification through Enzymolysis

The saccharification of cellucose through enzymolysis can be efficiently and quickly carried out with low enzyme consumption and low energy consumption by using the dual body circulatory reactor for cellulose enzymolysis of the invention to produce sugar for fermentation.

The cellulase for enzymolysis is not important for the present invention. The cellulase used in the present invention is not particularly limited, and can be various conventional cellulases in the art. These enzymes can be prepared using conventional methods or obtained through commercial channels.

Cellulase is also called the cellulase system, is a complex enzyme system composed of multiple components, and is a generic term of a group of enzymes for the hydrolysis of cellulose and the derivatives thereof. The typical and widely studied cellulose-producing strains mainly are filamentous fungus, wherein the cellulose-producing strains having high activity and whole enzyme system mainly includes: *Trichoderma* sp., *Penicillium* sp. and *Basidiomycetes*, such as *T. reesei*, *T. Koningii*, and *T. viride* of *Trichoderma* sp., and *P. decumbens*, *P. janthinellum* and *P.funiculosum* of *Penicillium* sp.

According to the substrates of cellulase, action site and released products, cellulase can be divided into three categories:
(1) endo-β-1,4-D-glucanase (1,4-D-glucanohydrolase or endo-1,4-β-D-glucanase, EC 3.2.1.4, EG), the molecular weight of which is 23-146 KDa. Such enzyme can act on the amorphous region in the cellulose molecules and randomly hydrolyze β-1,4-glycosidic bond, thereby cutting the long-chain fiber molecules to produce a large number of small cellulose molecules with non-reducing ends, such as short-chain cellulose, fiber oligosaccharides, etc. For example, the molecular weight of fungal isomerase EG I is 53 KDa, that of EG III is about 49.8 KDa, and that of EG for two kinds of *Cgtophaga* is only 6.3 KDa.
(2) exo-β-1,4-D-glucanase, or called as cellobiohydrolas (1,4-β-D-glucan cellobiohydrolase or exo-1,4-β-D-glucanase, EC 3.2.1.91, CBH), the molecular weight of which is 38-118 KDa. Such enzyme can act on the reducing or non-reducing ends of cellulose molecules, hydrolyze β-1,4-glycosidic bond and cut one cellobiose molecule every time to destroy cellulose molecules. For example, there are two kinds of CBH in *Trichoderma* sp., wherein the molecular weight of CBH I is about 66 KDa, and that of CBH II is about 53 KDa.
(3) β-1,4-glucosidase (EC 3.2.1.21, GE) including intracellular (or cell wall) enzyme and extracellular enzyme, the molecular weight of which is 47-76 KDa (extracellular enzyme) or 90-100 KDa (intracellular enzyme). This enzyme is mainly used for the catalytic hydrolysis of compounds comprising β-glucoside bonds to break non-reducing β-D-glucose residues and ends thereof and release β-D-glucose and corresponding ligands, such as aryl, hydroxyl and the like. In the process of cellulose hydrolysis, β-glucosidase can hydrolyze low molecular weight of cellodextrin and cellobiose to produce glucose, thereby relieving the inhibition on the activity of cellulase caused by the accumulation of cellobiose. Therefore, β-glucosidase is one of the main components of generalized cellulase system and the activity thereof can directly affect the effect of whole cellulase system.

In addition to the above three categories, some short peptide compounds, "hydrogen bond enzymes", "cellobiose dehydrogenase" and the like in fungus are involved in the degradation process of cellulose. They degrade cellulose molecules by oxidation or destroying the hydrogen bonds.

There is no specific limit to the amount of enzyme. Usually, the amount of enzyme can be 100-5000 IU/ml, preferably, 500-2000 IU/ml.

A particularly preferable enzyme is acidic cellulase, the amount of which is usually 100-5000 IU/ml, preferably, 200-3000 IU/ml, more preferably, 500-1500 IU/ml.

### The production of ethanol by fermentation

The sugar obtained from the degradation of cellulose using the method according to the invention can be used as the raw sugar materials for various fermentation processes, especially, for fermentation process for producing ethanol.

The fermentation processes for producing ethanol using various engineering bacterium are well-known in the art. The sugar prepared from the degradation of kenaf cellulose using the method according to the invention can be used in these processes. Briefly, the sugar prepared by the method according to the invention can be used in some or all of the fermentation processes.

The main advantages of the invention include:
(a) the saccharification through enzymolysis according to the present invention has many advantages, such as high conversion rate, short treatment time, high sugar conversion rate and low energy consumption;
(b) the enzyme consumption in the invention is decreased significantly;
(c) the invention is suitable for high volume reaction system for cellulose enzymolysis; and
(d) the whole efficiency of the invention is high and the cost is low.

The invention will be further illustrated with reference to the following specific examples. It is to be understood that these examples are only intended to illustrate the invention, but not to limit the scope of the invention. For the experimental methods in the following examples without particular conditions, they are performed under routine conditions or as instructed by the manufacturer.

Unless otherwise specified, all percentages, ratios, proportions or parts are by weight.

### General method

### Pretreatment

### The pretreatment of the fiber of kenaf:

The bast fiber of kenaf is treated to remove impurities such as gum, wax, some lignin and so on, and then washed in water at the temperature of 30-90°C. The slurry solution with good flowability is obtained by steam explosion.

### Example 1

### Saccharification through Enzymolysis

The saccharification through enzymolysis was carried out according to the following steps by using the equipment for the saccharification through enzymolysis shown in fig. 1.

### 1. steps:

1.1 preparing 100 L solution with 6% of substrate concentration from slurry solution obtained by pretreatment;
1.2 dissolving an appropriate amount of citric acid and trisodium citrate in the solution, and adjusting pH value to 4.8;
1.3 heating the jacket of dual body circulation reactor for enzymolysis to 50°C;
1.4 adding 75% of the total amount of cellulase, which can be a liquid enzyme and/or solid enzyme, into the solution, when the time was set as initial reaction time, t0, wherein the total amount of cellulase (including endoglucanase, exoglucanase and beta-glucosidase) was 1000 IU/ml. The slurry solution was loaded into the first reaction tank with the rotation speed being 600 rpm, and then loaded into the second reaction tank from the first reaction tank, wherein the rotation speed of the second reaction tank was 50 rpm. All of the material flowed circularly between dual bodies, i.e. the first reaction tank and the second reaction tank.
1.5 adding 15% of the total amount of cellulase which can be a liquid enzyme and/or solid enzyme into the solution after 24 hr;
1.6 adding 10% of the total amount of cellulase which can be a liquid enzyme and/or solid enzyme into the solution after 48 hr;
1.7 after 72 hr, enzymolysis reaction was quenched.

### 2. Results

Before enzymolysis, the content of cellulose in the pretreated raw materials is more than 65%.

After tested, the results of enzymolysis include:
98.96% of the conversion rate of reducing sugar in enzymatic hydrolysate;
91.04% of the conversion rate of glucose in enzymatic hydrolysate.

### Example 2

The saccharification through enzymolysis was carried out according to the following steps by using the equipment for the saccharification through enzymolysis shown in fig. 1.

### 1. steps:

1.1 preparing 100 L solution with 6% of substrate concentration from slurry solution obtained by pretreatment;
1.2 dissolving an appropriate amount of citric acid and trisodium citrate in the solution, and adjusting pH value to 4.8;
1.3 heating the jacket of dual body circulation reactor for enzymolysis to 50°C;
1.4 adding 75% of the total amount of cellulase, which can be a liquid enzyme and/or solid enzyme, into the solution, when the time was set as initial reaction time, t0, wherein the total amount of cellulase (including endoglucanase, exoglucanase and beta-glucosidase) was 900 IU/ml. The slurry solution was loaded into the first reaction tank with the rotation speed being 1500 rpm, and then loaded into the second reaction tank from the first reaction tank. The rotation speed of the second reaction tank was 120 rpm. All of the material flowed circularly between dual bodies, i.e. the first reaction tank and the second reaction tank.
1.5 adding 15% of the total amount of cellulase which can be a liquid enzyme and/or solid enzyme into the solution after 24 hr;
1.6 adding 10% of the total amount of cellulase which can be a liquid enzyme and/or solid enzyme into the solution after 48 hr;
1.7 after 60 hr, enzymolysis reaction was quenched.

### 2. Results

Before enzymolysis, the content of cellulose in the pretreated raw materials is more than 65%.

After tested, the results of enzymolysis include:
99.39% of the conversion rate of reducing sugar in enzymatic hydrolysate;
92.36% of the conversion rate of glucose in enzymatic hydrolysate.

### Example 3

The saccharification through enzymolysis was carried out according to the following steps by using the equipment for the saccharification through enzymolysis shown in fig. 1.

### 1. steps:

1.1 preparing 100 L solution with 6% of substrate concentration from slurry solution obtained by pretreatment;
1.2 dissolving an appropriate amount of citric acid and trisodium citrate in the solution, and adjusting pH value to 4.8;
1.3 heating the jacket of dual body circulation reactor for enzymolysis to 50°C;
1.4 adding 75% of the total amount of cellulase, which can be a liquid enzyme and/or solid enzyme, into the solution, when the time was set as initial reaction time, t0, wherein the total amount of cellulase (including endoglucanase, exoglucanase and beta-glucosidase) was 1200 IU/ml. The slurry solution was loaded into the first reaction tank with the rotation speed being 2800 rpm, and then loaded into the second reaction tank with the rotation speed being 250 rpm from the first reaction tank. All of the material flowed circularly between dual bodies, i.e. the first reaction tank and the second reaction tank.
1.5 adding 25% of the total amount of cellulase which can be a liquid enzyme and/or solid enzyme into the solution after 24 hr;
1.6 after 48 hr, enzymolysis reaction was quenched.

### 2. Results

Before enzymolysis, the content of cellulose of the pretreated raw material is more than 65%.

After tested, the results of enzymolysis include:
99.8% of the conversion rate of reducing sugar in enzymatic hydrolysate;
92.80% of the conversion rate of glucose in enzymatic hydrolysate.

### Example 4

### Fermentation experiment

High temperature resistant and high active dry yeast (*Saccharomyces cerevisiae*, bought from Hubei Angel Yeast co., LTD) was used.

The nutrient ingredients in Angel yeast fermentation medium include: yeast powder 0.5%, peptone 0.3%, (NH₄)₂SO₄ 0.1%, potassium dihydrogen phosphate 0.2%, and pH was adjusted to 5.0 by acetic acid.

The preparation of yeast starter: 0.5 g Angel high active dry yeast was dissolved in 2% of aqueous glucose solution. After shaken to uniform, the mixture was placed into the shaking table and cultured for 15-20 min at 35-40°C at 100 rpm. And then the temperature was reduced to below 34°C. After activated 1-2 hr, the mixture can be used as the yeast starter.

The fermentable sugar prepared in example 3 was used as raw materials. The fermentation was carried out using conventional conditions for fermentation, wherein the fermentation temperature was 35°C, the amount of yeast was 0.5%, and the fermentation time was 48 h.

### Results:

After tested, the concentration of ethanol in the raw liquid for fermentation is 9.44 g/L.

### Comparative example 1

Example 1 was repeated, and the difference is that the dual body circulation reactor for cellulose enzymolysis shown in fig. 1 was replaced by the dual body circulation reactor comprising two "the first reaction tanks" shown in fig. 1.

### Results:

Before enzymolysis, the content of cellulose of the pretreated raw material is more than 65%.

After tested, the results of enzymolysis include:
50.21% of the conversion rate of reducing sugar in enzymatic hydrolysate;
56.20% of the conversion rate of glucose in enzymatic hydrolysate.

### Comparative example 2

Example 2 was repeated, the difference is that the dual body circulation reactor for cellulose enzymolysis shown in fig. 1 was replaced by the dual body circulation reactor comprising two "the second reaction tanks" shown in fig. 1. Furthermore, in order to flow in the second reaction tank, the slurry solution was pretreated by using independent high-speed shear homogenizer at 1500 rpm for 15 min, and then loaded into the reactor.

### Results:

Before enzymolysis, the content of cellulose of the pretreated raw materials (before homogenized through shear) is more than 65%.

After tested, the results of enzymolysis include:
62.02% of the conversion rate of reducing sugar in enzymatic hydrolysate;
65.15% of the conversion rate of glucose in enzymatic hydrolysate.

It can be seen from examples 1-3 and comparative examples 1-2 that using the dual body circulation reactor for cellulose enzymolysis of the invention, conversion rate of the saccharification through enzymolysis can be improved significantly, the time for treatment is short, the conversion rate of sugar is high, and the enzyme consumption is decreased significantly, thereby reducing the cost of production.

### Example 4

The saccharification through enzymolysis was carried out according to the following steps by using the equipment for the saccharification through enzymolysis shown in fig. 5 and fig. 6.1.

### steps:

1.1 preparing 50 ton solution with 6% of substrate concentration from slurry solution obtained by pretreatment;
1.2 dissolving an appropriate amount of citric acid and trisodium citrate in the solution, and adjusting pH value to 4.8;
1.3 heating the jacket of dual body circulation reactor for enzymolysis to 50 °C;
1.4 adding 75% of the total amount of cellulase, which can be a liquid enzyme and/or solid enzyme, into the solution, when the time can be set as initial reaction time, t0, wherein the total amount of cellulase (including endoglucanase, exoglucanase and beta-glucosidase) was 1200 IU/ml. The slurry solution was loaded into the first reaction tank in which three shear machines were located in different depth and operated at 2800 rpm, and then loaded into six "the second reaction tanks" connected in series with the rotation speed being 50 rpm from the first reaction tank. All of the material flowed circularly between dual bodies, i.e. the first reaction tank and the second reaction tank.
1.5 adding 25% of the total amount of cellulase which can be a liquid enzyme and/or solid enzyme into the solution after 24 hr;
1.6 after 60 hr, enzymolysis reaction was quenched.

### 2. Results:

Before enzymolysis, the content of cellulose in the pretreated raw material is more than 65%.

After tested, the results of enzymolysis include:
98.8% of the conversion rate of reducing sugar in enzymatic hydrolysate;
91.04% of the conversion rate of glucose in enzymatic hydrolysate.

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A reactor for cellulose enzymolysis, wherein it comprises:
(a) a first reaction tank comprising a tank body, a shear homogenizer located in the tank body, an inlet for feeding, and an outlet for discharging a material;
(b) a second reaction tank comprising two or more reaction tubes connected in series or in parallel, wherein each reaction tube comprises a tube body, a rotating shaft and stirring impeller located in the tube body, an inlet, an outlet, and a thermal insulation layer located in the outside of the tube body; and the second reaction tank further comprises a motor for rotating the rotating shaft in the tube body;
(c) a pipeline connecting the first and the second reaction tanks, wherein said pipeline comprises a pipeline for transferring the material processed in the first reaction tank to the second reaction tank, and a pipeline for transferring the material processed in the second reaction tank to the first reaction tank; andeach pipeline can be equipped with an optional pipeline pump; and
(d) a pipeline connecting each tube body in the second reaction tank, wherein each pipeline can be equipped with an optional pipeline pump.

2. The reactor according to claim 1, wherein the volume of the first reaction tank is 3-150 t, and preferably 30-100 t.

3. The reactor according to claim 2, wherein the number of said shear homogenizer in the tank body is at least 2.

4. The reactor according to claim 3, wherein the first reaction tank further includes a tank cap rotating around the center axis and being located on the top of the tank body, and the shear homogenizer is fixed in the tank top.

5. The reactor according to claim 4, wherein the rotation speed of the tank cap rotating around the center axis and being located on the top of the tank body is 1-20 rpm, and preferably 2-10 rpm.

6. The reactor according to claim 3 or 4, wherein said shear homogenizers can be distributed with a staggered manner in the tank body of the first reaction tank.

7. The reactor according to claim 1, wherein the rotation speed of said rotating shaft located in the tube body in the second reaction tank can be 50-300 rpm, and preferably 100-250 rpm.

8. The reactor according to claim 1, wherein said rotating shaft in the second reaction tank is equipped with blades which form a helix angle of 15-45° to flow direction.

9. The reactor according to claim 1, wherein the depth-diameter aspect ratio of the second reaction tank body is 1: 0.5-1: 0.1, and preferably, 1: 0.25.

10. The reactor according to claim 1, wherein the rotation speed of the shear homogenizer located in the first reaction tank is 500-4000 rpm, and preferably, 1200-3000 rpm.

11. The reactor according to claim 1, wherein the ratio of the volume of the first reaction tank to that of the second reaction tank is 0.1: 1-1: 0.1, and preferably, 1: 0.5, wherein the volume of the second reaction tank is the sum of the volume of each tube body.

12. A method for the saccharification of cellulose through enzymolysis, wherein it comprises the following steps:
(a) homogenizing a cellulose raw material in the first reaction tank through shear force to form a homogenized cellulose raw material;
(b) hydrolyzing the homogenized cellulose raw material by using enzymes in the second reaction tank to degrade cellulose into oligosaccharide or monosaccharide, wherein the second reaction tank comprises two or more reaction tubes connected in series or in parallel, and in step b), the cellulose material self-circulates between or among the reaction tubes and the material exteriorly circulates between the first reaction tank and the second reaction tank.
